# EUROPEAN PATENT SPECIFICATION

(11) **EP 3 424 498 B1**
(45) Date of publication and mention of the grant of the patent: **28.04.2021**
(21) Application number: 17759209.4
(22) Date of filing: 28.02.2017
(51) Int. Cl.: A61K 9/51, A61K 47/32, A61K 39/395, A61P 35/00

(54) **TUMOUR THERAPEUTIC MONOCLONAL ANTIBODY NANO-CAPSULE AND PREPARATION METHOD AND USE THEREOF**
NANOKAPSEL MIT MONOKLONALEM ANTIKÖRPER FÜR TUMORTHERAPIE SOWIE HERSTELLUNGSVERFAHREN UND VERWENDUNG DAVON
NANOCAPSULE D'ANTICORPS MONOCLONAL THÉRAPEUTIQUE ANTITUMORAL, SON PROCÉDÉ DE PRÉPARATION ET UTILISATION

(30) Priority: 03.03.2016 CN 201610120570
(43) Date of publication of application: 09.01.2019
(73) Proprietor: Tianjin Nanuo Bio-Technology Co., Ltd., Tianjin 321001 (CN)
(72) Inventor: YUAN, Xubo, Tianjin 300073 (CN); KANG, Chunsheng, Tianjin 300022 (CN); LU, Yunfeng, Los Angeles California (US)
(74) Representative: Ter Meer Steinmeister & Partner
(86) International application number: PCT/CN2017/075103
(87) International publication number: WO 2017/148358

(56) References cited:
- CN-A- 104 208 684
- CN-A- 105 663 084
- MA MING ET AL: "Silica-coated magnetite nanoparticles labeled by nimotuzumab, a humanised monoclonal antibody to epidermal growth factor receptor: preparations, specific targeting and bioimaging.", JOURNAL OF NANOSCIENCE AND NANOTECHNOLOGY OCT 2013, vol. 13, no. 10, October 2013 (2013-10), pages 6541-6545, XP002794732, ISSN: 1533-4880
- LIANG SHENG ET AL: "Phosphorylcholine polymer nanocapsules prolong the circulation time and reduce the immunogenicity of therapeutic proteins", NANO RESEARCH, TSINGHUA UNIVERSITY PRESS, CN, vol. 9, no. 4, 1 February 2016 (2016-02-01), pages 1022-1031, XP035958780, ISSN: 1998-0124, DOI: 10.1007/S12274-016-0991-3 [retrieved on 2016-02-01]
- LU , SHOUTAO et al.: "Application Research Progress on 2-Methacryloyloxyethyl phosphorylcholine Copolymers", ENGINEERING PLASTICS APPLICATION, vol. 40, no. 8, 1 August 2012 (2012-08-01) , pages 1-4, XP055575965, ISSN: 1001-3539, DOI: 10.3969/j.issn.1001-3539.2012.08.024

## Description

### TECHNICAL FIELD

The invention belongs to the field of pharmaceutical preparation containing antigen or antibody, in particular is directed to a nimotuzumab nanocapsule encapsulated by poly-2-methacryloyloxyethyl phosphorylcholine and a preparation method and use thereof.

### BACKGROUND OF THE INVENTION

Glioblastoma is the most common primary malignant tumor in brain and is highly invasive. Currently, its treatment includes surgical resection, along with adjuvant radiotherapy and chemotherapy with temozolomide after resection, but with a survival period usually less than 15 months.

In recent years, with understanding of the mechanism of tumor onset and development and advancement in molecular biology technology, molecular targeted therapies targeting cell receptors, key genes and regulatory molecules have been initiated in clinical practice. Among them, hotspots are mainly targeting epidermal growth factor receptor (EGFR). EGFR is a transmembrane glycoprotein, consisting of an extracellular ligand binding domain, a transmembrane domain and an intracellular tyrosine kinase domain. Upon ligand binding to the extracellular ligand binding domain, the receptor homodimerizes or heterodimerizes, leading to activation of intracellular tyrosine kinase and in turn of downstream signaling pathways. The signaling mechanism of EGFR is intimately relevant to malignant phenotypes of tumors, inhibition on normal apoptosis, angiogenesis, and tumor metastasis, suggesting that EGFR can be used as a therapeutic target for drugs. By now, there have been three monoclonal antibodies against EGFR marketed in the world, which have achieved significant effects. The three antibodies are Nimotuzumab, Cetuximab, and Panitumumab. Nimotuzumab is a humanized IgG1 monoclonal antibody against EGFR. By binding to epitope 3A of the extracellular domain of EGFR, Nimotuzumab competitively inhibits the binding of ligand to EGFR, thereby inactivating the receptor, and achieving tumor-inhibiting effects. However, therapeutic antibodies suffer from defects such as large molecular weights, low efficiency in crossing the blood-brain barrier, poor stability and short circulation time in blood, and the like.

With the development and maturity of nanotechnology, many nanoscale carriers have been used for tumor targeted drug delivery, for example, nanocapsules, liposomes, nanoemulsion, polymer micelles, dendrimers, and the like. Among them, the nanocapsule refers to a microcapsule having a particle diameter of 10 to 1000 nm obtained by encapsulation of a solid or liquid drug by pharmaceutical excipient. The nanocapsule selectively releases the drug to tumor site by passive targeting and/or active targeting. Actively targeting nanocapsules, which target specific receptors on tumor cell surfaces or specifically expressed enzymes in tumor cells, can increase the concentration of anti-tumor drugs in tumor cells, thereby playing a better role than passively targeting nanocapsules.

Matrix metalloproteinases (MMPs) are zinc-dependent endopeptidases that utilize metal ions as enzyme cofactors to degrade various extracellular matrix proteins and process certain biologically active molecules. Since the discovery of the first MMP, collagenase MMP-1, in 1962, 26 members have been discovered to date. Among the MMP family members, MMP-2 and MMP-9 are important members known to be implicated in cancer metastasis and invasion. MMP-2 and MMP-9 are highly expressed in metastatic tumor cells and endothelial cells in blood vessels of tumor tissues. Therefore, MMP-2 and MMP-9 have attracted the attention of researchers as targets for tumor treatment and anti-tumor drug delivery systems.

Generally, the preparation of nanocapsules is mostly carried out by polymer dispersion method or monomer polymerization method. In recent years, in situ polymerization has evolved from nanocomposites. The reaction mechanism of the method is that, reactive monomers are adsorbed electrostatically to the surface of macromolecular drug, and then polymerized on the surface of the drug in the presence of catalyst.

### SUMMARY OF THE INVENTION

To overcome the defects of current tumor therapeutic antibody drugs, such as short half-life in blood, immunogenicity, and large molecular weight, and difficult to pass through the blood-brain barrier, the present invention provides a nimotuzumab nanocapsule and preparation method and use thereof.

The present invention is achieved in accordance with the following technical solutions.

The present invention provides a nimotuzumab nanocapsule having a brain tumor targeting delivery effect, which is prepared by polymerization of poly-2-methacryloyloxyethyl phosphorylcholine and nimotuzumab, and has a round and smooth surface, a uniform particle size with particle diameter of 30±5 nm and surface charge of 2.3 mV

The present invention provides a method for preparing nimotuzumab nanocapsules including the following steps:
a. taking a solution containing 0.1-100 mg nimotuzumab;
b. adding a monomer having a carbon-carbon double bond in a molar ratio of nimotuzumab to the monomer having a carbon-carbon double bond of 1:100-1:100000;
c. adding an enzyme degradable polypeptide crosslinker in a molar ratio of nimotuzumab to the crosslinker of 1:100-1:10000, to gather around nimotuzumab the monomer having a carbon-carbon double bond and the crosslinker by electrostatic interaction and hydrogen bonding;
d. adding an initiator in a molar ratio of nimotuzumab to the initiator of 1:100-1:10000, and performing in situ polymerization in the presence of the initiator at 4 °C for 0.1-24 hours, to obtaining poly-2-methacryloyloxyethyl phosphorylcholine encapsulated nimotuzumab nanocapsules.

Preferably, the monomer having a carbon-carbon double bond in step b consists of N-(3-aminopropyl)methacrylate and 2-methacryloyloxyethyl phosphorylcholine in a molar ratio of 0.0001:1-1:10000.

More preferably, the molar ratio of N-(3-aminopropyl)methacrylate to 2-methacryloyloxyethyl phosphorylcholine is 1:5-20.

Further preferably, the molar ratio of N-(3-aminopropyl)methacrylate to 2-methacryloyloxyethyl phosphorylcholine is 1:10-15.

Preferably, the crosslinker in step c is selected from the group consisting of an enzyme sensitive crosslinker, a pH sensitive crosslinker, or a redox sensitive crosslinker.

The enzyme sensitive crosslinker is matrix metalloproteinase-2 degradable polypeptide crosslinker; the pH sensitive crosslinker is ethylene glycol dimethacrylate; and the redox sensitive crosslinker is a disulfide-containing crosslinker.

More preferably, in step c, the crosslinker is matrix metalloproteinase-2 degradable polypeptide crosslinker.

Further preferably, in step c, the molar ratio of nimotuzumab to matrix metalloproteinase-2 degradable polypeptide crosslinker is 1:400-600, more preferably 1:450-550.

The initiator in step d consists of ammonium persulfate and tetramethylethylenediamine in a molar ratio of 0.01:1-1:100.

Further preferably, in step d, the molar ratio of ammonium persulfate to tetramethylethylenediamine is 1:1-5, more preferably 1:1.5-2.5.

As a preferred embodiment, the present invention provides a method for preparing nimotuzumab nanocapsules including the following steps:
a. taking a solution containing 1 mg nimotuzumab;
b. adding electropositive monomer N-(3-aminopropyl)methacrylate in a molar ratio of nimotuzumab to N-(3-aminopropyl)methacrylate of 1:300, then adding 2-methacryloyloxyethyl phosphorylcholine in a molar ratio of nimotuzumab to 2-methacryloyloxyethyl phosphorylcholine of 1:4000;
c. adding matrix metalloproteinase-2 degradable polypeptide crosslinker in a molar ratio of nimotuzumab to the crosslinker of 1:500, and letting stand for 10 minutes, to gather around nimotuzumab N-(3-aminopropyl)methacrylate, 2-methacryloyloxyethyl phosphorylcholine and the enzyme degradable polypeptide crosslinker by electrostatic interaction and hydrogen bonding;
d. adding ammonium persulfate and tetramethylethylenediamine in a molar ratio of nimotuzumab to ammonium persulfate to tetramethylethylenediamine of 1:500:1000, and reacting at 4 °C for 2 hours, to obtain poly-2-methylacryloyloxyethyl phosphorylcholine encapsulated nimotuzumab nanocapsules.

The present invention also provides a nimotuzumab nanocapsule prepared by the method above described, with nimotuzumab encapsulated in a shell formed by polymerization of a monomer having a carbon-carbon double bond and an enzyme-degradable crosslinker.

Preferably, the present invention provides a nimotuzumab nanocapsule prepared by the method above described, with nimotuzumab encapsulated in a shell formed by polymerization of N-(3-aminopropyl)methacrylate, 2-methacryloyloxyethyl phosphorylcholine and matrix metalloproteinase-2 degradable polypeptide crosslinker.

The present invention provides the use of the above described nimotuzumab nanocapsule in preparing a tumor targeted therapeutic drug.

The present invention provides the use of the above described nimotuzumab nanocapsule in preparing a drug for treating a brain tumor.

The present invention provides the use of the above described nimotuzumab nanocapsule in preparing a drug for treating glioma, particularly glioblastoma.

The present invention provides the use of the above described nimotuzumab nanocapsule in preparing a drug for treating gastric cancer.

The present invention achieves the following beneficial effects.

In the present invention poly-2-methacryloyloxyethyl phosphorylcholine encapsulated nimotuzumab nanocapsules are prepared by in-situ polymerization method. The nimotuzumab nanocapsules have uniform sizes, and round and smooth surfaces, as well as properties of tumor targeting, release by enzymatic degradation and long blood circulation time (half-life time in blood is 48 hours). The present nimotuzumab nanocapsules overcome the defect of low efficiency of nimotuzumab in passing through the blood-brain barrier, and achieve targeting and efficient delivery of nimotuzumab to brain tumors and gastric tumors, avoid toxic side effects of nimotuzumab to normal organs, and effectively inhibit the proliferation of glioma cells and gastric tumors utilizing anti-tumor effects of nimotuzumab, and finally improve anti-tumor efficacy of nimotuzumab, especially on brain malignant tumors. The in situ polymerization method used by the present invention is similar to bulk polymerization with simple operation and pure polymer output, while the reaction of the present method occurs in aqueous phase, which overcomes the problem of heat dissipation of the bulk polymerization. The method and nimotuzumab nanocapsules of the present invention have advantages of low cost, simple operation, good stability, good sustained release effect, and easy production in a large-scale industrialization, thus the present invention has a broad application prospect.

### BRIEF DESCRIPTION OF THE FIGURES

Figure 1 shows the transmission electron micrograph of the poly-2-methacryloyloxyethyl phosphorylcholine encapsulated nimotuzumab nanocapsule of the present invention;
Figure 2 shows the particle size distribution diagram of the poly-2-methacryloyloxyethyl phosphorylcholine encapsulated nimotuzumab nanocapsule of the present invention;
Figure 3 shows the zeta potential distribution diagram of the poly-2-methacryloyloxyethyl phosphorylcholine encapsulated nimotuzumab nanocapsule of the present invention;
Figure 4 shows detection results showing brain targeting of the poly-2-methacryloyloxyethyl phosphorylcholine encapsulated nimotuzumab nanocapsule of the present invention in an orthotopic nude mouse model of U87 glioma;
Figure 5 shows efficacy evaluation results of the poly-2-methacryloyloxyethyl phosphorylcholine encapsulated nimotuzumab nanocapsule of the present invention in an orthotopic nude mouse model of U87 glioma;
Figure 6 shows efficacy evaluation results of the poly-2-methacryloyloxyethyl phosphorylcholine encapsulated nimotuzumab nanocapsule of the present invention in a subcutaneous transplant nude mouse model of MGC803 gastric cancer.

### DETAILED DESCRIPTION OF PREFERRED EMBODIMENTS

The present invention will be further described below in conjunction with the figures and embodiments.

### Example 1 Preparation and characterization of poly-2-methacryloyloxyethyl phosphorylcholine encapsulated nimotuzumab nanocapsules

A solution containing 1 mg nimotuzumab (Baitai Biological Pharmaceutical Co., Ltd.) was taken, into which electropositive monomer N-(3-aminopropyl)methacrylate was added in the molar ratio of nimotuzumab to N-(3-aminopropyl)methacrylate of 1:300, followed by the addition of 2-methacryloyloxyethyl phosphorylcholine in the molar ratio of nimotuzumab to 2-methacryloyloxyethyl phosphorylcholine of 1:4000; and then matrix metalloproteinase-2 degradable polypeptide crosslinker was add in the molar ratio of nimotuzumab to the crosslinker of 1:500. The reaction system was allowed to stand for 10 minutes, allowing the reactive monomers and the enzyme degradable polypeptide crosslinker to gather around nimotuzumab by electrostatic interaction and hydrogen bonding. Then ammonium persulfate and tetramethylethylenediamine were added in the molar ratio of nimotuzumab to ammonium persulfate to tetramethylethylenediamine of 1:500:1000, the reaction system was kept at 4 °C for 2 hours, and poly-2-methylacryloyloxyethyl phosphorylcholine encapsulated nimotuzumab nanocapsules were obtained.

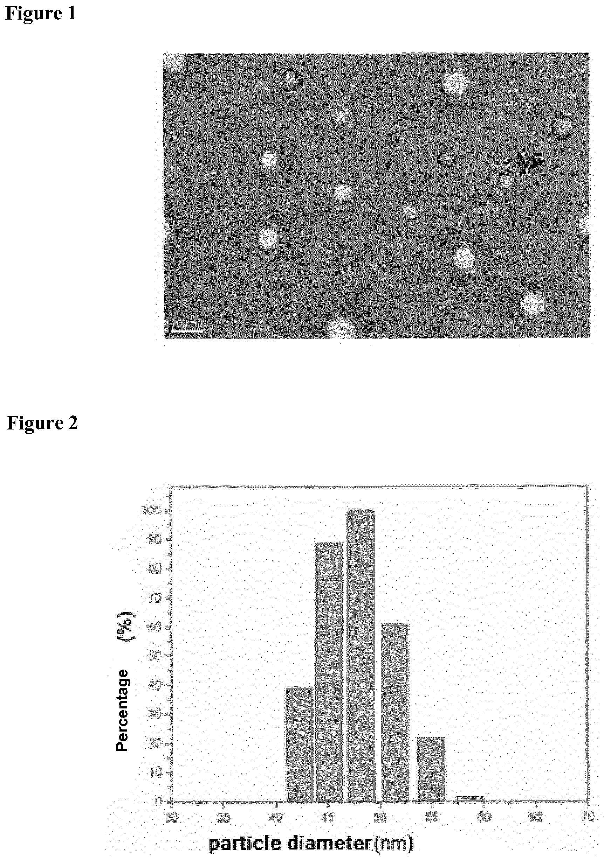

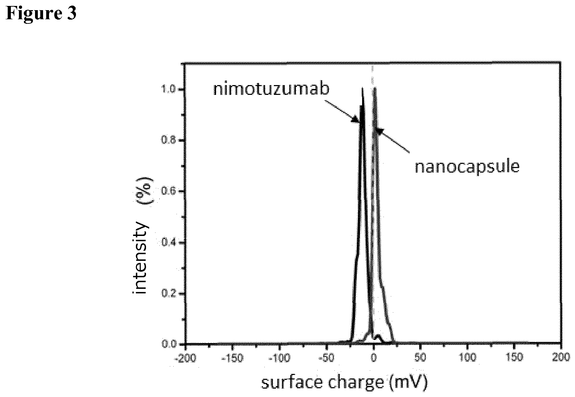

The poly-2-methacryloyloxyethyl phosphorylcholine encapsulated nimotuzumab nanocapsules were observed under a transmission electron microscope. As shown in Fig. 1, the nanocapsules had round and smooth surfaces and uniform particle size. The particle size distribution and surface charge of the obtained nanocapsules were further detected with a particle size analyzer (BI-90Plus, Brookhaven Instruments, USA). It can be seen that the nanocapsules had round and smooth surfaces and uniform particle size with particle diameter of 30±5 nm (as shown in Fig. 2), and surface charge of 2.3 mV (as shown in Fig. 3).

### Example 2 Efficacy Evaluation of poly-2-methacryloyloxyethyl phosphorylcholine encapsulated nimotuzumab nanocapsules in an orthotopic nude mouse model of U87 glioma

On the day before virus infection, U87 cells (ATCC, USA; HTB-14) were plated on a 24-well plate at a density of 1×10⁵-1×10⁶, cultured at 5% CO₂ and 37°C in DMEM (GBICO, USA; 11965-092) supplemented with imported fetal bovine serum (HyClone; SH30071.03). After 24 hours, 10-500 µl of viral solution encoding luciferase (Shanghai GenePharma Co., Ltd.) was added together with 10-50 µl of hexadimethrine bromide (polybrene) to increase infection efficiency. 12 hours later, the culture medium was replaced with DMEM; after 48 hours, 10-100 µl Puromycin, the selection drug was added, and after selection for 2 weeks, positive clones were obtained and propagated for use in transplantation into the brain of nude mouse to establish an orthotopic model.

When nude mice were anaesthetized and stabilized, a surgical incision was made at the top of head of each of the nude mice and a hole was made on the skull. Then the animals were placed in a stereotactic instrument (Stoelting, USA; lab standard™) individually; the two cells above described were digested and re-suspended in an appropriate amount of medium, and slowly injected into the brain of each mouse with a syringe; and then the skin was sutured with operative suture. 10 days after tumor implantation, the tumor formation rate was observed by in vivo imaging. Animals were randomly grouped with 10 mice in each group, and injected in tail vein nimotuzumab or poly-2-methacryloyloxyethyl phosphorylcholine encapsulated nimotuzumab nanocapsules respectively at a dose of 5 mg/kg body weight every other day for total of 10 times. The intracranial tumor size of the animals was monitored every 10 days using an in vivo imaging system (Xenogen, Waltham, MA, USA; 200), and the survival periods of the animals were counted. The statistical results of survival periods were: control vs antibody treatment group, p= 0.1745; control vs nanocapsule treatment group, p=0.0385; antibody treatment group vs nanocapsule treatment group, p=0.0061.

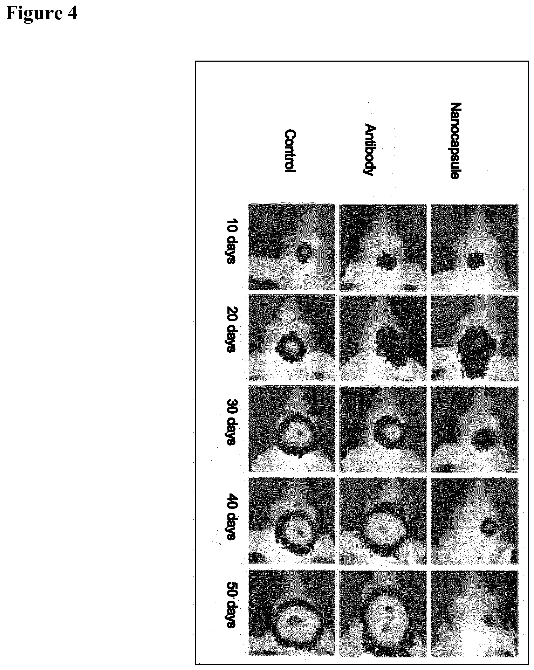

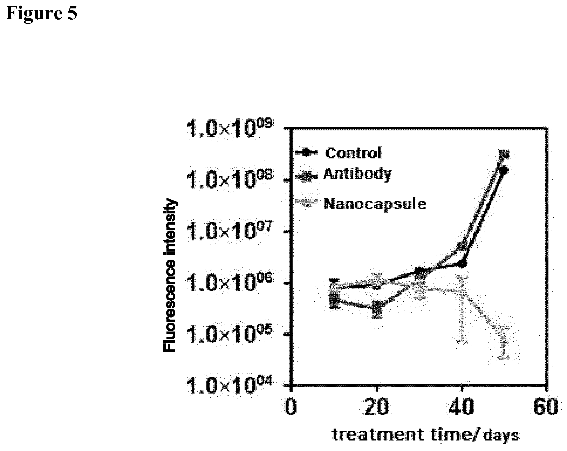

As shown in Fig. 4 and Fig. 5, compared with the antibody treatment group, the brain tumor growth rate in animals in the group treated with poly-2-methacryloyloxyethyl phosphorylcholine encapsulated nimotuzumab nanocapsules was significantly slowed down, which was further confirmed by the quantitative detection results of tumor size at five observation points (10, 20, 30, 40, 50 days) after the treatment started.

### Example 3 Efficacy Evaluation of poly-2-methacryloyloxyethyl phosphorylcholine encapsulated nimotuzumab nanocapsules in subcutaneous transplant nude mouse model of MGC803 gastric cancer

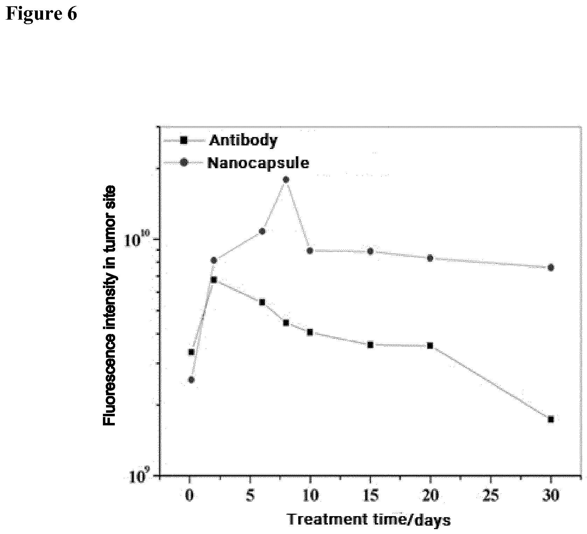

MGC803 gastric cancer cells stably expressing luciferase were inoculated subcutaneously into a 4 week old nude mouse at a dose of 5 x 10⁵ cells per injection point using a 100 µl microinjector to establish a tumor source. When the subcutaneous tumor reached a long diameter of 3 cm, tumor blocks were removed, evenly chopped, and implanted subcutaneously into nude mice in respective experimental groups (n=6). The animals stayed on feeding, and when the long diameter of tumor mass reached about 5 mm, the experimental groups began to receive treatment. Administration scheme was injection in tail vein at a dose of 5 mg/kg body weight for once. At the same time, the therapeutic effect was observed by collecting activity value of the luciferase in the tumor cells using an in vivo imaging system every two days for 30 days, and then the nude mice in each treatment group were sacrificed and the tumor masses were peeled off. As shown in Fig. 6, compared with the antibody treatment group, the tumor growth rate of the poly-2-methacryloyloxyethyl phosphorylcholine encapsulated nanocapsule treatment group was significantly slowed down.

## Claims

1. A nimotuzumab nanocapsule having a brain tumor targeting delivery effect, which is prepared by polymerization of poly-2-methacryloyloxyethyl phosphorylcholine and nimotuzumab, and has a round and smooth surface, a uniform particle size with particle diameter of 30±5 nm and surface charge of 2.3 mV.

2. A method for preparing nimotuzumab nanocapsules including the following steps:
a. taking a solution containing 0.1-100 mg nimotuzumab;
b. adding a monomer having a carbon-carbon double bond in a molar ratio of nimotuzumab to the monomer having a carbon-carbon double bond of 1:100-1:100000;
c. adding an enzyme-degradable polypeptide crosslinker in a molar ratio of nimotuzumab to the crosslinker of 1:100-1:10000, to gather around nimotuzumab the monomer having a carbon-carbon double bond and the crosslinker by electrostatic interaction and hydrogen bonding;
d. adding an initiator in a molar ratio of nimotuzumab to the initiator of 1:100-1:10000, and performing in situ polymerization in the presence of the initiator at 4 °C for 0.1-24 hours, to obtain poly-2-methacryloyloxyethyl phosphorylcholine encapsulated nimotuzumab nanocapsules.

3. The method for preparing nimotuzumab nanocapsules according to claim 2, wherein the monomer having a carbon-carbon double bond in step b consists of N-(3-aminopropyl)methacrylate and 2-methacryloyloxyethyl phosphorylcholine in a molar ratio of 0.0001:1-1:10000;
preferably, the molar ratio of N-(3-aminopropyl)methacrylate to 2-methacryloyloxyethyl phosphorylcholine is 1:5-20;
more preferably, the molar ratio of N-(3-aminopropyl)methacrylate to 2-methacryloyloxyethyl phosphorylcholine is 1:10-15.

4. The method for preparing nimotuzumab nanocapsules according to claim 2, wherein the crosslinker in step c is selected from the group consisting of an enzyme sensitive crosslinker, a pH sensitive crosslinker, or a redox sensitive crosslinker;
preferably, the enzyme sensitive crosslinker is a matrix metalloproteinase-2 degradable polypeptide crosslinker; the pH sensitive crosslinker is ethylene glycol dimethacrylate; and the redox sensitive crosslinker is a disulfide-containing crosslinker.

5. The method for preparing nimotuzumab nanocapsules according to claim 4, wherein in step c, the crosslinker is matrix metalloproteinase-2 degradable polypeptide crosslinker;
preferably, the molar ratio of nimotuzumab to matrix metalloproteinase-2 degradable polypeptide crosslinker is 1:400-600, more preferably 1:450-550.

6. The method for preparing nimotuzumab nanocapsules according to claim 2, wherein the initiator in step d consists of ammonium persulfate and tetramethylethylenediamine in a molar ratio of 0.01:1-1:100;
preferably, the molar ratio of ammonium persulfate to tetramethylethylenediamine is 1:1-5, more preferably 1:1.5-2.5.

7. A method for preparing nimotuzumab nanocapsules including the following steps:
a. taking a solution containing 1 mg nimotuzumab;
b. adding electropositive monomer N-(3-aminopropyl)methacrylate in a molar ratio of nimotuzumab to N-(3-aminopropyl)methacrylate of 1:300, then adding 2-methacryloyloxyethyl phosphorylcholine in a molar ratio of nimotuzumab to 2-methacryloyloxyethyl phosphorylcholine of 1:4000;
c. adding matrix metalloproteinase-2 degradable polypeptide crosslinker in a molar ratio of nimotuzumab to the crosslinker of 1:500, and letting stand for 10 minutes to gather around nimotuzumab N-(3-aminopropyl)methacrylate, 2- methacryloyloxyethyl phosphorylcholine and the enzyme degradable polypeptide crosslinker by electrostatic interaction and hydrogen bonding;
d. adding ammonium persulfate and tetramethylethylenediamine in a molar ratio of nimotuzumab to ammonium persulfate to tetramethylethylenediamine of 1:500:1000, and reacting at 4 °C for 2 hours, to obtain poly-2-methylacryloyloxyethyl phosphorylcholine encapsulated nimotuzumab nanocapsules.

8. A nimotuzumab nanocapsule prepared by the method according to any one of claims 2 to 7, wherein nimotuzumab is encapsulated in a shell formed by polymerization of a monomer having a carbon-carbon double bond and an enzyme-degradable crosslinker.

9. The nimotuzumab nanocapsule according to claim 8, wherein nimotuzumab is encapsulated in a shell formed by polymerization of N-(3-aminopropyl)methacrylate, 2-methacryloyloxyethyl phosphorylcholine and matrix metalloproteinase-2 degradable polypeptide crosslinker.

10. The nimotuzumab nanocapsule according to any one of claims 1, 8 and 9 for use in treating tumor; preferably, the nimotuzumab nanocapsule for use in treating a brain tumor;
more preferably, the nimotuzumab nanocapsule for use in treating glioma, particularly glioblastoma;
further preferably, the nimotuzumab nanocapsule for use in treating gastric cancer.

## Patentansprüche

1. Nimotuzumab-Nanokapsel mit einem Hirntumor-Zielabgabeeffekt, die durch Polymerisation von Poly-2-methacryloyloxyethylphosphorylcholin und Nimotuzumab hergestellt wird und eine runde und glatte Oberfläche, eine einheitliche Teilchengröße mit einem Teilchendurchmesser von 30±5 nm und eine Oberflächenladung von 2,3 mV aufweist.

2. Verfahren zur Herstellung von Nimotuzumab-Nanokapseln, umfassend die folgenden Schritte:
a. Verwenden einer Lösung, die 0,1-100 mg Nimotuzumab enthält;
b. Zugabe eines Monomers mit einer Kohlenstoff-Kohlenstoff-Doppelbindung in einem molaren Verhältnis von Nimotuzumab zu dem Monomer mit einer Kohlenstoff-Kohlenstoff-Doppelbindung von 1:100-1:100000;
c. Hinzufügen eines enzymabbaubaren Polypeptid-Vernetzers in einem molaren Verhältnis von Nimotuzumab zu dem Vernetzer von 1:100-1:10000, um das Monomer mit einer Kohlenstoff-Kohlenstoff-Doppelbindung und den Vernetzer durch elektrostatische Wechselwirkung und Wasserstoffbrückenbindung um Nimotuzumab herum anzusammeln;
d. Zugeben eines Initiators in einem molaren Verhältnis von Nimotuzumab zu dem Initiator von 1:100-1:10000 und Durchführen einer in situ-Polymerisation in Gegenwart des Initiators bei 4 °C für 0,1-24 Stunden, um Nimotuzumab-Nanokapseln zu erhalten, die in Poly-2-methacryloyloxyethylphosphorylcholin eingekapselt sind.

3. Verfahren zur Herstellung von Nimotuzumab-Nanokapseln nach Anspruch 2, wobei das Monomer mit einer Kohlenstoff-Kohlenstoff-Doppelbindung in Schritt b aus N-(3-Aminopropyl)methacrylat und 2-Methacryloyloxyethylphosphorylcholin in einem molaren Verhältnis von 0,0001:1-1:10000 besteht;
vorzugsweise beträgt das molare Verhältnis von N-(3-Aminopropyl)methacrylat zu 2-Methacryloyloxyethylphosphorylcholin 1:5-20;
weiter bevorzugt ist das molare Verhältnis von N-(3-Aminopropyl)methacrylat zu 2-Methacryloyloxyethylphosphorylcholin 1:10-15.

4. Verfahren zur Herstellung von Nimotuzumab-Nanokapseln nach Anspruch 2, wobei der Vernetzer in Schritt c ausgewählt ist aus der Gruppe bestehend aus einem enzymsensitiven Vernetzer, einem pH-sensitiven Vernetzer oder einem redoxsensitiven Vernetzer; vorzugsweise ist der enzymsensitive Vernetzer ein durch Matrix-Metalloproteinase-2 abbaubarer Polypeptid-Vernetzer; der pH-sensitive Vernetzer ist Ethylenglykoldimethacrylat; und der redoxsensitive Vernetzer ist ein Disulfid-haltiger Vernetzer.

5. Verfahren zur Herstellung von Nimotuzumab-Nanokapseln nach Anspruch 4, wobei in Schritt c der Vernetzer ein durch Matrix-Metalloproteinase-2 abbaubarer Polypeptid-Vernetzer ist;
vorzugsweise beträgt das molare Verhältnis von Nimotuzumab zu Matrix-Metalloproteinase-2 abbaubarem Polypeptid-Vernetzer 1:400-600, mehr bevorzugt 1:450-550.

6. Verfahren zur Herstellung von Nimotuzumab-Nanokapseln nach Anspruch 2, wobei der Initiator in Schritt d aus Ammoniumpersulfat und Tetramethylethylendiamin in einem molaren Verhältnis von 0,01:1-1:100 besteht;
vorzugsweise beträgt das molare Verhältnis von Ammoniumpersulfat zu Tetramethylethylendiamin 1:1-5, besonders bevorzugt 1:1,5-2,5.

7. Verfahren zur Herstellung von Nimotuzumab-Nanokapseln, umfassend die folgenden Schritte:
a. Verwenden einer Lösung, die 1 mg Nimotuzumab enthält;
b. Zugabe des elektropositiven Monomers N-(3-Aminopropyl)methacrylat in einem molaren Verhältnis von Nimotuzumab zu N-(3-Aminopropyl)methacrylat von 1:300, dann Zugabe von 2-Methacryloyloxyethylphosphorylcholin in einem molaren Verhältnis von Nimotuzumab zu 2-Methacryloyloxyethylphosphorylcholin von 1:4000;
c. Zugabe von Matrix-Metalloproteinase-2-abbaubarem Polypeptid-Vernetzer in einem molaren Verhältnis von Nimotuzumab zu dem Vernetzer von 1:500 und Stehenlassen für 10 Minuten, um N-(3-Aminopropyl)methacrylat, 2-Methacryloyloxyethylphosphorylcholin und den Enzym-abbaubaren Polypeptid-Vernetzer durch elektrostatische Wechselwirkung und Wasserstoffbrückenbindung um Nimotuzumab anzusammeln;
d. Zugabe von Ammoniumpersulfat und Tetramethylethylendiamin in einem molaren Verhältnis von Nimotuzumab zu Ammoniumpersulfat zu Tetramethylethylendiamin von 1:500:1000 und Umsetzen bei 4 °C für 2 Stunden, um Nimotuzumab-Nanokapseln zu erhalten, die in Poly-2-methylacryloyloxyethylphosphorylcholineingekapselt sind.

8. Nimotuzumab-Nanokapsel, hergestellt nach dem Verfahren nach einem der Ansprüche 2 bis 7, wobei Nimotuzumab in einer Hülle eingekapselt ist, die durch Polymerisation eines Monomers mit einer Kohlenstoff-Kohlenstoff-Doppelbindung und einem enzymabbaubaren Vernetzer gebildet wird.

9. Nimotuzumab-Nanokapsel nach Anspruch 8, wobei Nimotuzumab in einer Hülle eingekapselt ist, die durch Polymerisation von N-(3-Aminopropyl)methacrylat, 2-Methacryloyloxyethylphosphorylcholin und einem durch Matrix-Metalloproteinase-2 abbaubaren Polypeptid-Vernetzer gebildet wird.

10. Nimotuzumab-Nanokapsel nach einem der Ansprüche 1, 8 und 9 zur Verwendung bei der Behandlung eines Tumors;
vorzugsweise die Nimotuzumab-Nanokapsel zur Verwendung bei der Behandlung eines Gehirntumors;
weiter bevorzugt, die Nimotuzumab-Nanokapsel zur Verwendung bei der Behandlung eines Glioms, insbesondere eines Glioblastoms;
weiter bevorzugt, die Nimotuzumab-Nanokapsel zur Verwendung bei der Behandlung von Magenkrebs.

## Revendications

1. Nanocapsule de nimotuzumab ayant un effet de libération ciblant les tumeurs cérébrales, qui est préparée par polymérisation de poly-2-méthacryloyloxyéthylphosphorylcholine et de nimotuzumab, et qui a une surface ronde et lisse, une taille de particules uniforme avec un diamètre de particules de 30±5 nm et une charge de surface de 2,3 mV.

2. Procédé de préparation de nanocapsules de nimotuzumab comprenant les étapes suivantes :
a. prendre une solution contenant 0,1-100 mg de nimotuzumab ;
b. ajouter un monomère ayant une double liaison carbone-carbone dans un rapport molaire du nimotuzumab au monomère ayant une double liaison carbone-carbone de 1:100-1:100000 ;
c. ajouter un agent de réticulation polypeptidique dégradable par une enzyme dans un rapport molaire du nimotuzumab à l'agent de réticulation de 1:100-1:10000, pour rassembler autour du nimotuzumab le monomère ayant une double liaison carbone-carbone et l'agent de réticulation par interaction électrostatique et liaison hydrogène ;
d. ajouter un initiateur dans un rapport molaire de nimotuzumab à l'initiateur de 1:100-1:10000, et effectuer une polymérisation in situ en présence de l'initiateur à 4 °C pendant 0,1-24 heures, pour obtenir des nanocapsules de nimotuzumab encapsulées dans du poly-2-méthacryloyloxyéthylphosphorylcholine.

3. Procédé de préparation de nanocapsules de nimotuzumab selon la revendication 2, dans lequel le monomère ayant une double liaison carbone-carbone dans l'étape b est constitué de N-(3-aminopropyl)méthacrylate et de 2-méthacryloyloxyéthylphosphorylcholine dans un rapport molaire de 0,0001:1-1:10000;
de préférence, le rapport molaire du N-(3-aminopropyl)méthacrylate à la 2-méthacryloyloxyéthylphosphorylcholine est de 1:5-20 ;
plus préférablement, le rapport molaire du N-(3-aminopropyl)méthacrylate à la 2-méthacryloyloxyéthylphosphorylcholine est de 1:10-15.

4. Procédé de préparation de nanocapsules de nimotuzumab selon la revendication 2, dans lequel l'agent de réticulation de l'étape c est choisi dans le groupe constitué par un agent de réticulation sensible aux enzymes, un agent de réticulation sensible au pH ou un agent de réticulation sensible à l'oxydoréduction ;
de préférence, l'agent de réticulation sensible aux enzymes est un agent de réticulation polypeptidique dégradable par la métalloprotéinase-2 de la matrice ; l'agent de réticulation sensible au pH est le diméthacrylate d'éthylène glycol ; et l'agent de réticulation sensible à l'oxydoréduction est un agent de réticulation contenant un disulfure.

5. Procédé de préparation de nanocapsules de nimotuzumab selon la revendication 4, dans lequel, dans l'étape c, l'agent de réticulation est un agent de réticulation polypeptidique dégradable par la métalloprotéinase-2 de la matrice ;
de préférence, le rapport molaire du nimotuzumab à l'agent de réticulation polypeptidique dégradable par la métalloprotéinase-2 de la matrice est de 1:400-600, plus préférablement de 1:450-550.

6. Procédé de préparation de nanocapsules de nimotuzumab selon la revendication 2, dans lequel l'initiateur de l'étape d est constitué de persulfate d'ammonium et de tétraméthyléthylènediamine dans un rapport molaire de 0,01:1-1:100 ;
de préférence, le rapport molaire du persulfate d'ammonium à la tétraméthyléthylènediamine est de 1:1-5, plus préférablement de 1:1,5-2,5.

7. Procédé de préparation de nanocapsules de nimotuzumab comprenant les étapes suivantes :
a. prendre une solution contenant 1 mg de nimotuzumab ;
b. ajouter le monomère électropositif de N-(3-aminopropyl)méthacrylatedans un rapport molaire de nimotuzumab au N-(3-aminopropyl)méthacrylate de 1:300, puis ajouter de la 2-méthacryloyloxyéthylphosphorylcholine dans un rapport molaire de nimotuzumab à la 2-méthacryloyloxyéthylphosphorylcholine de 1:4000 ;
c. ajouter un agent de réticulation polypeptidique dégradable par la métalloprotéinase-2 de la matrice dans un rapport molaire de nimotuzumab à l'agent de réticulation de 1:500, et laisser reposer pendant 10 minutes pour rassembler autour du nimotuzumab le N-(3-aminopropyl)méthacrylate, la 2-méthacryloyloxyéthylphosphorylcholine et le agent de réticulation polypeptidique dégradable par une enzyme par interaction électrostatique et liaison hydrogène ;
d. ajouter du persulfate d'ammonium et de tétraméthyléthylènediamine dans un rapport molaire de nimotuzumab au persulfate d'ammonium et à la tétraméthyléthylènediamine de 1:500:1000, et réaction à 4 °C pendant 2 heures, pour obtenir des nanocapsules de nimotuzumab encapsulées dans du poly-2-méthacryloyloxyéthylphosphorylcholine.

8. Nanocapsule de nimotuzumab préparée par le procédé selon l'une quelconque des revendications 2 à 7, dans laquelle le nimotuzumab est encapsulé dans une enveloppe formée par polymérisation d'un monomère ayant une double liaison carbone-carbone et un agent de réticulation dégradable par une enzyme.

9. Nanocapsule de nimotuzumab selon la revendication 8, dans laquelle le nimotuzumab est encapsulé dans une enveloppe formée par polymérisation de N-(3-aminopropyl)méthacrylate, de 2-méthacryloyloxyéthylphosphorylcholine et d'un agent de réticulation polypeptidique dégradable par la métalloprotéinase-2 de la matrice.

10. Nanocapsule de nimotuzumab selon l'une des revendications 1, 8 et 9 pour utilisation dans le traitement des tumeurs ;
de préférence, la nanocapsule de nimotuzumab pour le traitement d'une tumeur cérébrale ;
plus préférablement, la nanocapsule de nimotuzumab pour le traitement d'un gliome, en particulier d'un glioblastome ;
de préférence, la nanocapsule de nimotuzumab pour le traitement du cancer de l'estomac.
